# EUROPÄISCHE PATENTSCHRIFT

(11) **EP 0 795 533 B1**
(45) Veröffentlichungstag und Bekanntmachung des Hinweises auf die Patenterteilung: **31.05.2000**
(21) Anmeldenummer: 97103609.0
(22) Anmeldetag: 05.03.1997
(51) Int. Cl.: C07C 45/50, C07C 45/78, B01J 31/40

(54) **Verfahren zur Herstellung von Aldehyden mittels eines Rhodium und substituierte Diphenyldiphosphane enthaltenden Katalysatorsystems**
Process for the preparation of aldehydes using a catalyst system containing rhodium and substituted diphenyldiphosphanes
Procédé pour la préparation d'aldéhydes à l'aide d'un système catalytique contenant du rhodium et des diphényldiphosphanes substitués

(30) Priorität: 11.03.1996 DE 19609337
(43) Veröffentlichungstag der Anmeldung: 17.09.1997
(73) Patentinhaber: Celanese Chemicals Europe GmbH, 60439 Frankfurt am Main (DE)
(72) Erfinder: Bahrmann, Helmut, Dr., 46499 Hamminkeln (DE); Kleiner, Hans-Jerg Dr., 61476 Kronberg (DE); Regnat, Dieter, Dr., 65817 Eppstein (DE)

(56) Entgegenhaltungen:
- EP-A- 0 354 588
- EP-A- 0 374 615
- EP-A- 0 621 075
- GB-A- 1 266 180

## Beschreibung

Die vorliegende Erfindung betrifft ein Verfahren zur Herstellung von Aldehyden durch Umsetzung von olefinischen Verbindungen mit Wasserstoff und Kohlenmonoxid bei erhöhtem Druck in homogener Phase und in Gegenwart eines Rhodium sowie neue substituierte Diphenyldiphosphane enthaltenden Katalysatorsystems und die Abtrennung des Reaktionsproduktes vom Katalysator.

Es ist bekannt, durch Umsetzung von Olefinen mit Kohlenmonoxid und Wasserstoff Aldehyde und Alkohole herzustellen. Die Reaktion wird durch Hydridometallcarbonyle, vorzugsweise solcher der Metalle der 8. Gruppe des Periodensystems, katalysiert. Neben Kobalt, das als Katalysatormetall in großem Umfang technische Anwendung findet, hat in letzter Zeit Rhodium zunehmende Bedeutung erlangt. Im Gegensatz zu Kobalt gestattet Rhodium, die Reaktion bei niedrigem Druck durchzuführen; darüber hinaus werden vorzugsweise geradkettige n-Aldehyde und nur in untergeordnetem Maße iso-Aldehyde gebildet. Schließlich ist auch die Hydrierung der Olefine zu gesättigten Kohlenwasserstoffen bei Anwendung von Rhodium-Katalysatoren deutlich niedriger als bei Anwendung von Kobalt-Katalysatoren.

Bei den in der Technik eingeführten Verfahren wird der Rhodium-Katalysator in Form von modifizierten Hydridorhodiumcarbonylen eingesetzt, die zusätzliche und gegebenenfalls im Überschuß eingesetzte Liganden enthalten. Besonders bewährt haben sich als Liganden tertiäre Phosphine oder Phosphite. Ihre Anwendung ermöglicht, den Reaktionsdruck auf Werte unter 30 MPa zu senken.

Probleme wirft bei diesem Verfahren jedoch die Abtrennung der Reaktionsprodukte und die Wiedergewinnung der im Reaktionsprodukt homogenen gelösten Katalysatoren auf. Im allgemeinen destilliert man hierzu das Umsetzungsprodukt aus dem Reaktionsgemisch ab. In der Praxis kann dieser Weg wegen der thermischen Empfindlichkeit der gebildeten Aldehyde und Alkohole aber nur bei der Hydroformylierung niedriger Olefine, d.h. Olefine mit bis zu etwa 5 Kohlenstoffatomen im Molekül, beschritten werden.

Bei der Hydroformylierung langkettiger Olefine oder olefinischer Verbindungen mit funktionellen Gruppen werden Produkte mit hohem Siedepunkt gebildet, die sich destillativ vom homogen gelösten Rhodium-Komplexkatalysator nicht abtrennen lassen. Die thermische Belastung des Destillationsgutes führt durch Dickölbildung zu erheblichen Verlusten an Wertprodukten und an Katalysator durch Zersetzung der Rhodiumkomplexverbindungen.

Die Abtrennung des Katalysators auf thermischem Wege wird durch Anwendung in Wasser löslicher Katalysatorsysteme vermieden. Derartige Katalysatoren sind z.B. in der DE-PS 26 27 354 beschrieben. Die Löslichkeit der Rhodiumkomplexverbindungen wird hierbei durch Verwendung von sulfonierten Triarylphosphinen als Komplexbestandteil erreicht. Die Abtrennung des Katalysators vom Reaktionsprodukt nach Beendigung der Hydroformylierungsreaktion erfolgt bei dieser Verfahrensvariante einfach durch Trennung von wäßriger und organischer Phase, d.h. ohne Destillation und damit ohne zusätzliche thermische Verfahrensschritte. Ein weiteres Merkmal dieser Arbeitsweise ist, daß mit hoher Selektivität aus endständigen Olefinen n-Aldehyde und nur in ganz untergeordnetem Maße iso-Aldehyde gebildet werden. Neben sulfonierten Triarylphosphinen werden als Komplexbestandteile wasserlöslicher Rhodiumkomplexverbindungen auch carboxylierte Triarylphosphine eingesetzt.

Der Einsatz wasserlöslicher Katalysatoren hat sich ausgezeichnet bei der Hydroformylierung niederer Olefine, insbesondere Ethylen und Propylen, bewährt. Setzt man höhere Olefine wie Hexen, Octen oder Decen ein, gehen jedoch der Umsatz und/oder die Selektivität der Reaktion zu n-Verbindungen merklich zurück. Die Wirtschaftlichkeit der Umsetzung in technischem Maßstab ist dann häufig nicht mehr gegeben.

Eine weitere vorteilhafte Methode den Katalysator vom Produkt abzutrennen besteht darin, die metallorganischen Koordinationskomplexe aus organischen Flüssigkeiten durch eine selektiv wirkende Trennmembran auszusondern.

Ein derartiges Verfahren ist in EP 03 74 615 beschrieben. Die den Katalysator enthaltende organische Lösung wird mit einer semipermeablen Membran aus einem aromatischen Polyamid (Polyaramid) in Kontakt gebracht. Als treibende Kraft für den Trennvorgang kann hierbei sowohl eine Druckdifferenz (Druckfiltration) oder Konzentrationsdifferenz (Dialyse) dienen.

Als Liganden haben sich bei der Metallkomplex-katalysierten Hydroformylierung dreiwertige phosphororganische Verbindungen, insbesondere Phosphane, bewährt. Bei diesen Phosphanen handelt es sich um Monophosphane, die Sulfonat oder Carboxylat-Gruppen besitzen, also um anionische Phosphane. Die dazugehörigen Kationen leiten sich von sekundären oder tertiären Aminen, die langkettige Alkylreste tragen, ab.

Nachteilig bei Verwendung dieser Monophosphane als Katalysatorbestandteil ist, daß die Monophosphane in einem vergleichsweise hohen Überschuß, bezogen auf das Übergangsmetall, beispielsweise Rhodium, eingesetzt werden müssen. Das ist zum einen unökonomisch und führt zum anderen zu einer hohen Salzkonzentration im Reaktionsgemisch. Eine hohe Salzkonzentration kann unter Umständen die Umsetzung des Olefins mit Kohlenmonoxid und Wasserstoff ungünstig beeinflussen, da eine hohe Salzkonzentration die Löslichkeit der Reaktionspartner im Reaktionsgemisch beeinträchtigt und auch eine Schaumbildung begünstigen kann. Darüber hinaus wirkt sich eine hohe Salzkonzentration bei der Abtrennung des Katalysators ungünstig aus, weil sie im Falle einer destillativen Abtrennung zu einem erhöhten Anteil an salzhaltigem Dicköl führt oder im Falle einer Druckfiltration die Durchflußleistung der semipermeablen Membran in erheblichen Maße herabsetzt.

Es besteht daher ein Interesse, ein Verfahren zur Herstellung von Aldehyden bereitzustellen, das die vorstehend beschriebenen Nachteile nicht aufweist und sich ohne großen Aufwand realisieren läßt. Zudem soll das verwendete Katalysatorsystem eine recht hohe Aktivität besitzen und das gewünschte Wertprodukt in guter Ausbeute zugänglich machen. Darüber hinaus soll das Katalysatorsystem eine ausreichende Lebensdauer besitzen und sich nach Abtrennung aus dem Reaktionsgemisch für einen Wiedereinsatz eignen.

Gelöst wird diese Aufgabe durch ein Verfahren zur Herstellung von Aldehyden. Es ist dadurch gekennzeichnet, daß man eine olefinische Verbindung mit 2 bis 20 Kohlenstoffatomen mit Kohlenmonoxid und Wasserstoff in Anwesenheit oder Abwesenheit eines Lösungsmittels in Gegenwart eines Katalysators enthaltend Rhodium und eine Verbindung oder ein Gemisch von Verbindungen der allgemeinen Formel (I) 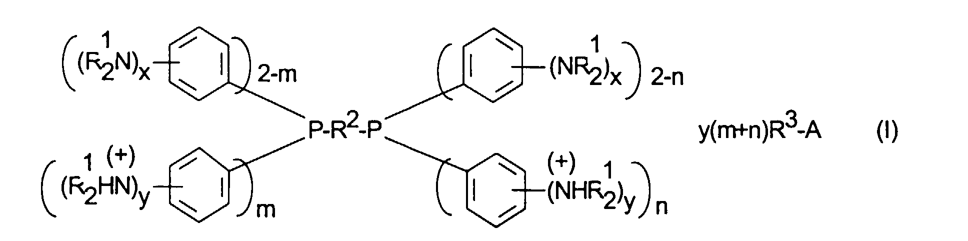 worin R¹ für H oder einen Alkylrest mit 1 bis 12 Kohlenstoffatomen, R² für einen geradkettigen Alkylenrest mit 1 bis 8 Kohlenstoffatomen, einen Sauerstoff enthaltenden Alkylenrest mit 2 bis 4 Kohlenstoffatomen, einen Rest der Formel (II) oder (III) 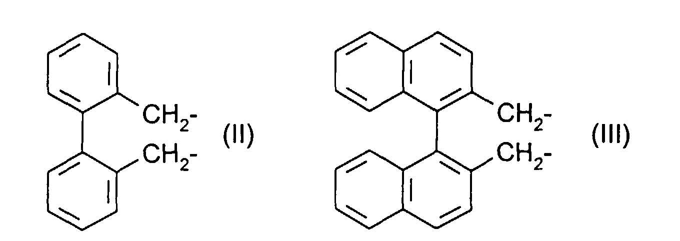 oder einen Cycloalkylenrest mit 3 bis 10 Kohlenstoffatomen, R³ für einen Alkylrest mit 1 bis 25 Kohlenstoffatomen oder einen Arylrest mit 6 bis 10 Kohlenstoffatomen steht, A einen Rest -COO⁻ oder -SO₃⁻ darstellt und x = 0, y = 1, m = 1 und n = 1 ist, oder x = 1, y = 1, m = (1 oder 2) und n = (1 oder 2) ist, oder, falls R² einen Rest der Formel (II) oder (III) darstellt, x = 1, y = 0, m = (0 oder 1) und n = (0 oder 1) ist, bei einer Temperatur von 50 bis 190°C und unter einem Druck von 0,1 bis 45 MPa umsetzt und das Reaktionsprodukt vom Katalysator abtrennt.

Ein Vorteil des erfindungsgemäßen Verfahrens besteht darin, daß es sich hinsichtlich der Abtrennung des Katalysators flexibel handhaben läßt. Man kann beispielsweise Aldehyde mit vergleichsweise niedrigen Siedepunkten durch Destillation abtrennen. Es ist allerdings auch möglich, den Katalysator durch Umsalzen in eine wasserlösliche Form zu überführen und ihn durch Extraktion in einer wäßrigen Phase abzutrennen. Eine weitere interessante Möglichkeit eröffnet sich, indem man den Katalysator auf eine besonders schonende Weise mittels einer Membranfiltration (Druckfiltration) aus dem Reaktionsprodukt abtrennt. Hierbei leitet man das Reaktionsprodukt unter Druck über eine geeignete Membran und trennt das hydroformylierte Produkt als Permeat ab, während der Katalysator und Ligand als Salze sich im Retentat stark anreichern.

Ein weiterer Vorteil des erfindundungsgemäßen Verfahrens ist, daß die Verbindung der Formel (I), die als Katalysatorbestandteil und gegebenenfalls als überschüssig eingesetzter Ligand dient, durch Variation der Reste R³-A und durch Variation der Art dieser Reste in ihren Eigenschaften beeinflußt werden kann. Langkettige oder voluminöse Reste R³ sind bei der Abtrennung des Katalysators mittels Membranfiltration von Vorteil, da sie das Rückhaltevermögen der Membran begünstigen und die Permeation durch die Membran herabsetzen.

Darüber hinaus gestattet das erfindungsgemäße Verfahren, mit vergleichsweise niedrigen Rhodiummengen und einem vergleichsweise niedrigem Verhältnis Verbindung der Formel (I): Rh zu arbeiten. Dies ist ein Beweis dafür, daß der Katalysator unter Reaktionsbedingungen eine hohe Reaktivität, aber zugleich auch eine hohe Stabilität besitzt. Diese vorteilhaften Eigenschaften bleiben auch, wie aus Beispiel 2 hervorgeht, beim Wiedereinsatz des gebrauchten, durch Membranfiltration abgetrennten Katalysators erhalten.

Üblicherweise setzt man in das erfindungsgemäße Verfahren, wie zuvor erwähnt, eine olefinische Verbindung mit 2 bis 20, insbesondere 6 bis 18, bevorzugt 8 bis 16 Kohlenstoffatomen, die eine-oder mehrere olefinische Doppelbindungen besitzen kann, ein. Die olefinische Verbindung kann aliphatisch, cycloaliphatisch oder araliphatisch sein.

Als Beispiele für aliphatische Verbindungen stehen geradkettige und/oder verzweigte Olefine mit endständiger und/oder innenständiger Lage der Doppelbindung. Besonders geeignet sind geradkettige Olefine mit 2 bis 20 Kohlenstoffatomen wie Ethen, Propen, Buten-1, Penten-1, n-Hexen-1, n-Hepten-1, n-Octen-1, n-Nonen-1, n-Decen-1, n-Undecen-1 und n-Dodecen-1, acyclische Terpene und verzweigte Olefine wie Diisobutylen, Tripropylen, Tetrapropylen und Dimersol.

Beispiele für aliphatische Diene sind 1,3-Butadien, 1,5-Hexadien und 1,9-Decadien. Beispiele für cycloaliphatische Einsatzstoffe sind Cyclohexen, Cycloocten, Cyclooctadien, Dicyclopentadien, cyclische Terpene, beispielsweise Limonen, Pinen, Camphoren und Bisabolen, insbesondere Dicyclopentadien. Beispielhaft für araliphatische Olefine steht Styrol.

Als olefinische Verbindungen mit funktionellen Gruppen sind Acrylsäurederivate, insbesondere die Ester, Methacrylsäurederivate, insbesondere die Ester, Allylverbindungen, insbesondere die Alkohole und Ester, Vinylverbindungen, insbesondere die Ester und Ether, Cyanoverbindungen, insbesondere Acrylnitril, sowie Acroleinderivate zu nennen.

Die vorstehend aufgeführten olefinischen Verbindungen stellen lediglich eine Auswahl dar, die, ohne einen Anspruch auf Vollständigkeit zu erheben, getroffen wurde.

Der Katalysator enthält Rhodium und eine Verbindung der Formel (I) oder ein Gemisch von Verbindungen der Formel (I). Diese Gemische der Verbindungen der Formel (I) bilden sich häufig im Verlauf der Herstellung, beispielsweise bei Einführen der Säurereste R³-A. Die Umsetzung des Amingruppen enthaltenden, noch nicht protonierten Bisphosphans mit den die Säurereste R³-A enthaltenden Säuren führt meist zu einem Gemisch entsprechender Salze.

Üblicherweise setzt man eine Verbindung der Formel (I), worin x = 0, y = 1, m = 1 und n = 1 ist, oder x = 1, y = 1, m = (1 oder 2) und n = (1 oder 2), insbesondere x = 1, y = 1, m = (1 oder 2) und n = (1 oder 2) ist, oder ein Gemisch dieser Verbindungen ein.

In der Verbindung der Formel (I) kann die R¹₂N- und R¹₂NR⁺-Gruppe in dem jeweiligen Benzolring an beliebiger Stelle stehen. Zweckmäßigerweise setzt man eine Verbindung der Formel (I), worin die R¹₂N- und die R¹₂NH⁺-Gruppe in meta- oder para-Stellung, insbesondere in para-Stellung zu der Bindung, die den Benzolring mit dem jeweiligen P-Atom verbindet, steht, oder ein Gemisch dieser Verbindungen ein.

Wegen ihrer relativ guten Zugänglichkeit und Verfügbarkeit setzt man eine Verbindung der Formel (I), worin R¹ für einen Alkylrest mit 1 bis 4 Kohlenstoffatomen, insbesondere für einen Methyl- oder Ethylrest, bevorzugt für einen Methylrest steht, oder ein Gemisch dieser Verbindungen ein.

Das Verfahren läßt sich mit gutem Erfolg unter Verwendung einer Verbindung der Formel (I), worin R² für einen Alkylenrest mit 1 bis 4 Kohlenstoffatomen oder einen Rest -(CH₂)₂-O-(CH₂)₂-, insbesondere für einen Trimethylen- oder Tetramethylenrest steht, oder unter Verwendung eines Gemisches dieser Verbindungen durchführen.

Es hat sich in einer Vielzahl von Fällen als günstig erwiesen, eine Verbindung der Formel (I), worin R³ für einen Alkylrest mit 12 bis 24 Kohlenstoffatomen oder einen Arylrest mit 6 bis 7 Kohlenstoffatomen, insbesondere für einen Alkylrest mit 14 bis 22 Kohlenstoffatomen steht, oder ein Gemisch dieser Verbindungen einzusetzen.

Wie eingangs bereits erwähnt, steht A für einen Rest COO⁻ oder SO₃⁻, insbesondere für einen Rest COO⁻. Es läßt sich auch ein Gemisch dieser Verbindungen der Formel (I) einsetzen.

Das erfindungsgemäße Verfahren läßt sich in einer Reihe von Fällen mit vergleichsweise niedrigen Mengen an Rhodium durchführen. Es kann aber auch mit relativ großen Mengen an Rhodium praktiziert werden. Im allgemeinen läßt sich die Umsetzung in Gegenwart von 5 bis 500, insbesondere 10 bis 150 ppm Rhodium, bezogen auf olefinische Verbindung, durchführen.

Das Verhältnis von Verbindung der Formel (I) zu Rhodium kann in recht weiten Grenzen variiert werden. Üblicherweise setzt man die Verbindung der Formel (I) und Rhodium in einem Verhältnis von 1:1 bis 200:1, insbesondere 2:1 bis 10:1 (Mol Verbindung der Formel (I) je g-Atom Rhodium) ein.

Zur Herstellung des Katalysators verwendet man üblicherweise eine Rhodiumverbindung, beispielsweise ein Rhodiumsalz. Geeignete Rhodiumsalze sind, ohne Anspruch auf Vollständigkeit zu erheben, Rhodiumchlorid, Rhodiumsulfat, Rhodiumnitrat, Rhodiumcarboxylate wie Rhodiumacetat, Rhodiumpropionat, Rhodiumbutyrat, Rhodium-2-ethylhexanoat.

Man bringt die Rhodiumverbindung mit der Verbindung der Formel (I) zusammen und stellt eine Lösung des Gemisches beider Stoffe her. Geeignet als Lösungsmittel sind organische Lösungsmittel, die unter den Bedingungen der Hydroformylierung inert sind. Hierzu zählen unter anderem aromatische Kohlenwasserstoffe, beispielsweise Toluol, ortho-Xylol, meta-Xylol, para-Xylol, Gemische isomerer Xylole, Ethylbenzol, Mesitylen, Gemische dieser Verbindungen, aliphatische Kohlenwasserstoffe wie Hexan, Octan oder auch cycloaliphatische Kohlenwasserstoffe wie Cyclohexan.

Es läßt sich jedoch auch die olefinische Verbindung und das Hydroformylierungsprodukt als Lösungsmittel für den Katalysator verwenden.

Man kann die Lösung der Rhodiumverbindung und der Verbindung der Formel (I) unmittelbar in die Hydroformylierung einsetzen oder den Katalysator in zuvor präformierter Form in die Umsetzung einsetzen.

Wie zuvor bereits erläutert, eignet sich das erfindungsgemäße Verfahren für die Hydroformylierung einer Vielzahl von olefinischen Verbindungen. Beispielsweise kann man als olefinische Verbindung ein substituiertes oder unsubstituiertes Alken mit 2 bis 20 Kohlenstoffatomen, ein substituiertes oder unsubstituiertes Dien mit 4 bis 10 Kohlenstoffatomen, ein substituiertes oder unsubstituiertes Cycloalken oder Dicycloalken mit 5 bis 12 Kohlenstoffatomen im Ringsystem, einen Ester einer ungesättigten Carbonsäure mit 3 bis 20 und eines aliphatischen Alkohols mit 1 bis 18 Kohlenstoffatomen, einen Ester einer gesättigten Carbonsäure mit 2 bis 20 Kohlenstoffatomen und eines ungesättigten Alkohols mit 2 bis 18 Kohlenstoffatomen, einen ungesättigten Alkohol oder Ether mit jeweils 3 bis 20 Kohlenstoffatomen oder ein araliphatisches Olefin mit 8 bis 20 Kohlenstoffatomen einsetzen.

Die Reaktionstemperatur hängt im gewissen Umfang auch von der Art der olefinischen Verbindung ab, die umgesetzt werden soll. Vergleichsweise reaktive olefinische Verbindungen lassen sich bei relativ niedrigen Temperaturen umsetzen, während vergleichsweise reaktionsträge olefinische Verbindungen relativ hohe Temperaturen erfordern.

Im allgemeinen genügt es, wie eingangs erwähnt, die Umsetzung bei einer Temperatur von 50 bis 190°C ablaufen zu lassen. In vielen Fällen hat es sich bewährt, die Umsetzung bei 80 bis 180°C, insbesondere 100 bis 170°C durchzuführen. Man kann auch bei Temperaturen unterhalb 50°C arbeiten, muß hierbei jedoch verlängerte Reaktonszeiten in Kauf nehmen. Es ist auch möglich, oberhalb von 190°C zu arbeiten, muß hierbei jedoch eventuell mit einer erhöhten Bildung von Nebenprodukten rechnen wie Hexan, Octan oder auch cycloaliphatische Kohlenwasserstoffe wie Cyclohexan.

Die Umsetzung läuft unter Druck in Gegenwart von Kohlenmonoxid und Wasserstoff ab. Kohlenmonoxid und Wasserstoff können im Molverhältnis 5:1 bis 1:5, insbesondere 2:1 bis 1:2, bevorzugt 1,2:1 bis 1:1,2 eingesetzt werden.

Häufig genügt es, die Umsetzung bei einem Druck von 1 bis 35 MPa, insbesondere 3 bis 30 MPa durchzuführen. Da ein hoher Druck die Umsetzung in der Regel begünstigt, wird man in einer Vielzahl von Fällen bei relativ hohen Drücken, beispielsweise oberhalb 15, insbesondere oberhalb 20 MPa arbeiten. Diese Arbeitsweise wird man bevorzugen, falls geeignete Druckbehälter zur Verfügung stehen.

Man kann das Verfahren in Anwesenheit oder Abwesenheit eines organischen Lösungsmittels, das unter den Bedingungen der Hydroformylierung inert ist, durchführen. Geeignete Lösungsmittel sind aromatische Kohlenwasserstoffe, beispielsweise Toluol, ortho-Xylol, meta-Xylol, para-Xylol, Gemische isomerer Xylole, Ethylbenzol, Mesitylen, aliphatische Kohlenwasserstoffe, beispielsweise Hexan, Octan oder auch cycloaliphatische Kohlenwasserstoffe und Gemische dieser Verbindungen.

Es ist jedoch in einer Reihe von Fällen möglich, auf die Verwendung eines organischen Lösungsmittels zu verzichten. Hierbei übernimmt die olefinische Verbindung und das gebildete Hydroformylierungsprodukt die Rolle eines Lösungsmittels.

Nach Beendigung der Umsetzung wird das Reaktionsprodukt gekühlt und durch Entspannen von gasförmigen Bestandteilen befreit. Anschließend trennt man das dabei anfallende Reaktionsgemisch mittels Destillation, Extraktion oder Druckfiltration vom Katalysator ab.

Welche Art der Abtrennung man wählt, hängt von der Art des Hydroformylierungprodukts ab. Hydroformylierungsprodukte, die thermisch relativ stabil sind und keine sehr hohen Siedepunkte besitzen, können destillativ, beispielsweise unter reduziertem Druck, vom Katalysator abgetrennt werden.

Eine extraktive Entfernung des Katalysators wird man bevorzugen, falls es möglich ist, den Katalysator mit einem vertretbaren Aufwand beispielsweise in eine wasserlösliche Form zu überführen und mittels einer Extraktion als wäßrige Phase abzutrennen. Auf diese Weise ist eine schonende Abtrennung des Katalysators aus dem Reaktionsgemisch zu erreichen. Aus der wäßrigen Phase läßt sich der Katalysator, gegebenenfalls durch Umsalzen, wieder in eine wasserunlösliche, für einen Wiedereinsatz geeignete Form umwandeln.

Nach einer besonderen Verfahrensvariante trennt-man das Reaktionsgemisch durch Druckfiltration mittels einer semipermeablen Membran, insbesondere mittels einer Polyamidmembran, bevorzugt mittels einer Polyaramidmembran ab. Hierbei leitet man das Reaktionsgemisch unter Druck, beispielsweise 1 bis 5 MPa, über die Membran und erhält ein weitestgehend vom Katalysator befreites Permeat, während der Katalysator sich im Retentat stark anreichert.

Die den Katalysator enthaltenden Retentate können vereinigt und, gegebenenfalls nach einer Präformierung und gegebenenfalls nach Ergänzung mit frischem Katalysator, wieder in das Verfahren eingesetzt werden. Ein mehrfacher Wiedereinsatz ist möglich, ohne nennenswerte Einbußen hinsichtlich Aktivität und Selektivität des Katalysators in Kauf nehmen zu müssen, wobei zumeist auf eine Präformierung (zusätzliche Behandlung mit Kohlenmonoxid und Wasserstoff) ebenso wie auf eine Zugabe von frischen Katalysator (Rhodium und/oder Verbindung der Formel (I)) verzichtet werden kann. Bei der Handhabung des Katalysators in aktivem Zustand ist generell unter Ausschluß von Luft zu arbeiten, da bereits sehr geringe Mengen Sauerstoff den Katalysator schädigen, respektive irreversibel desaktivieren. Hierfür ist in erster Linie vermutlich die Oxidation von P(III) zu P(V) in der Verbindung der Formel (I) verantwortlich.

Der Vollständigkeit halber sei an dieser Stelle darauf hingewiesen, daß die Verbindungen der Formel (I) sowie ihre Herstellung Gegenstand einer am gleichen Tag wie die vorliegende Patentanmeldung eingereichten deutschen Patentanmeldung (Aktenzeichen 196 093 36.8) sind, siehe auch EP-A-0 795 559.

Die nachfolgenden Beispiele belegen die Erfindung, ohne sie hierauf zu beschränken.

### Experimenteller Teil

### Beispiel 1

### Präformierung des Katalysators

In einer Glasvorlage werden unter Stickstoffatmosphäre 550 g von Sauerstoff befreites Toluol vorgelegt und unter Rühren mit 0,53 g 1,4-Bis-[bis-(4-dimethylaminophenyl)phosphino]butan x 2 Palmitinsäure und 0,19 mmol Rhodium, in Form von Rh-2-ethylhexanoat, versetzt. Die resultierende Toluol-Lösung wird unter Stickstoffatmosphäre in einen gründlich mit Stickstoff gespülten 5 l Autoklaven mit Rührwerk überführt. Man stellt unter Rühren durch Aufpressen von Synthesegas einen Druck von 270 bar [27 MPa] ein, heizt auf 125°C auf und läßt 2 Stunden reagieren. Danach ist die Präformierung des Katalysators abgeschlossen.

### Hydroformylierung

Nach Beendigung der Präformierung werden bei 270 bar [27 MPa] Druck und 125°C innerhalb von 1,5 Stunden insgesamt 1300 g Propylen aus einer Druckvorlage in den Autoklaven eingepumpt. Der Druck wird durch Aufpressen von Synthesegas bei 270 bar gehalten. Die Reaktionstemperatur beträgt 125°C, wobei die durch die Hydroformylierung freigesetzte, überschüssige Wärme mittels Kühlung (Luftgebläse) abgeführt wird. Die Hydroformylierung läuft in Gegenwart von 15 ppm Rhodium, bezogen auf gesamt eingesetztes Propylen, ab. Das Verhältnis 1,4-Bis-[bis-(4-dimethylaminophenyl)phosphino]butan x 2 Palmitinsäure zu Rhodium beträgt 2,5:1 (entsprechend einem Verhältnis P:Rh = 5:1). Man läßt nach Ende des Einpumpens noch 1 Stunde nachreagieren, kühlt anschließend den Autoklaven ab und entspannt ihn innerhalb von 1,5 Stunden.

Der Inhalt des Autoklaven wird mit dem verbleibenden Restdruck in einen 6 l Dreihalskolben mit Tauchstutzen entleert und gewogen (2630 g). Aus der Gewichtszunahme errechnet sich ein Propylenumsatz von 92 %. Unter Berücksichtigung der beim Entspannen des Autoklaven auftretenden Verluste entspricht dies einem nahezu vollständigen Umsatz.

| Das Rohprodukt enthält: | |
|---|---|
| 8,74 pm | Rh |
| 118 ppm | P (gesamt) |
| 1,6 mmol | P(III)/kg |
| 34,0 mg | gesamt basischer Stickstoff (in Eisessig)/kg |

### Membranfiltration (Druckfiltration)

2541 g des bei der Hydroformylierung erhaltenen Rohproduktes werden über eine Labormembranfilteranlage (M 577/579 = Bezeichnung ?) geführt. Als Membran wird eine Polyaramidmembran (VF-PA 5/PET 100; ein Produkt der Hoechst AG) verwendet, die vor Gebrauch 15 Minuten lang bei 100°C in Wasser getempert wird.
Mittels einer Umwälzpumpe wird die Membran mit 150 l/Stunde unter einem Druck von 15 bar [1,5 MPa] überströmt. 93 % des Rohprodukts passieren die Membran als Permeat. 149,2 g verbleiben als aufkonzentriertes Rhodium und das 1,4-Bis-[bis-(4-dimethylaminophenyl)phosphino]butan x 2 Palmitinsäure enthaltendes Retentat.

| Das Permeat enthält: | |
|---|---|
| 0,25 ppm | Rh |
| 15,0 ppm | P (gesamt) |
| 0,9 mmol | P(III)/kg |
| 16,0 mg | gesamt basischer Stickstoff/kg |

Die Flußleistung sinkt während der Membranfiltration (Druckfiltration) von anfangs 77,5 l/m² x Stunde auf 49,1 l/m² x Stunde. Das Absinken der Flußleistung ist durch die fortschreitende Aufkonzentrierung des Retentats bedingt.

Das gesamte Permeat wird einer zweiten Membranfiltration (Druckfiltration) unter den gleichen Bedingungen, wie zuvor beschrieben, unterworfen. Die Retentatmenge beträgt 141,6 g.

| Das Permeat enthält: | | Rückhalt* |
|---|---|---|
| 0,21 ppm | Rh | 97,65 % |
| 6,6 ppm | P (gesamt) | 94,41 % |
| 1,00 mmol | P(III)/kg | 37,5 % |
| 11,00 mg | gesamt basischer Stickstoff (in Eisessig)/ kg | |

| | | |
|---|---|---|
| * Der Rückhalt bezieht sich auf die Mengen der ursprünglich eingesetzten Stoffe. | | |

Die Flußleistung sinkt während der Membranfiltration (Druckfiltration) von anfangs 84 l/m² x Stunde auf 81,4 l/m² x Stunde.

### Beispiel 2

### Hydroformylierung unter Wiedereinsatz des Katalysators

Man legt die den Katalysator enthaltenden Retentate (149,2 g + 141,2 g aus Beispiel 1) unter Stickstoffatmosphäre vor, ergänzt mit Toluol auf insgesamt 550 g, stellt unter Rühren durch Aufpressen von Synthesegas einen Druck von 270 bar ein und heizt auf 125°C auf.

Anschließend führt man die Hydroformylierung unter Zugabe von 1300 g Propylen innerhalb von 1,5 Stunden wie in Beispiel 1 beschrieben durch. Das erhaltene Rohprodukt wird einer Membranfiltration (Druckfiltration) unterzogen und die vereinigten Retentate, die den Katalysator enthalten, werden wieder eingesetzt und die Hydroformylierung wird, wie zuvor beschrieben, mit 1300 g Propylen, die innerhalb von 1,5 Stunden zugepumpt werden, bei 270 bar Druck und 125°C durchgeführt. Aus der Gewichtszunahme des nach dem zweiten Wiedereinsatz des Katalysators (Retentate der Membranfiltration) erhaltenen Reaktionsproduktes errechnet sich ein Propylenumsatz von 95,6 %.

### Vergleichsbeispiel

### Präformierung des Katalysators

In einer Glasvorlage werden unter Stickstoffatmosphäre 550 g von Sauerstoff befreites Toluol vorgelegt und unter Rühren mit 0,249 g (0,95 mmol) Triphenylphosphin und 0,19 mmol Rhodium, in Form von Rh-2-ethylhexanoat, versetzt. Die resultierende Toluol-Lösung wird unter Stickstoffatmosphäre in einen gründlich mit Stickstoff gespülten 5 l Autoklaven mit Rührwerk überführt. Man stellt unter Rühren durch Aufpressen von Synthesegas einen Druck von 270 bar [27 MPa] ein, heizt auf 125°C auf und läßt 2 Stunden reagieren. Danach ist die Präformierung des Katalysators abgeschlossen.

### Hydroformylierung

Nach Beendigung der Präformierung werden bei 270 bar [27 MPa] Druck und 125°C innerhalb von 1,5 Stunden insgesamt 1300 g Propylen aus einer Druckvorlage in den Autoklaven eingepumpt. Der Druck wird durch Aufpressen von Synthesegas bei 270 bar gehalten. Die Reaktionstemperatur beträgt 125°C, wobei die durch die Hydroformylierung freigesetzte, überschüssige Wärme mittels Kühlung (Luftgebläse) abgeführt wird. Die Hydroformylierung läuft in Gegenwart von 15 ppm Rhodium, bezogen auf gesamt eingesetztes Propylen, ab. Das Verhältnis Triphenylphosphin zu Rhodium beträgt 5:1 (entsprechend einem Verhältnis P:Rh = 5:1). Man läßt nach Ende des Einpumpens noch 1 Stunde nachreagieren, kühlt anschließend den Autoklaven ab und entspannt ihn innerhalb von 1,5 Stunden.

Der Inhalt des Autoklaven wird mit dem verbleibenden Restdruck in einen 6 l Dreihalskolben mit Tauchstutzen entleert und gewogen (1704 g). Aus der Gewichtszunahme errechnet sich ein Propylenumsatz von 59 %.

Zum Vergleich sind in der nachfolgenden Tabelle die Reaktionsbedingungen und die Ergebnisse von Beispiel 1 unter Verwendung von 1,4-Bis-[bis-(4-dimethylaminophenyl)phosphinobutan x 2 Palmitinsäure und vom Vergleichsbeispiel unter Verwendung von Triphenylphosphin zusammengestellt.

**Tabelle**

| Hydroformylierung von Propylen | | |
|---|---|---|
| | Beispiel 1 | Vergleichsbeispiel |
| Propylen | 1300 g | 1300 g |
| Einpumpzeit | 1,5 h | 1,5 h |
| Rh* | 15 ppm | 15 ppm |
| Temperatur | 125°C | 125°C |
| Druck** | 270 bar | 270 bar |
| P:Rh | 5:1 | 5:1 |
| Reaktionsprodukt | 2630 g | 1704 g |
| n:i-Verhältnis*** | 62:38 | 60:40 |
| Umsatz (bezogen auf Propylen) | 92 % | 59 % |

| | | |
|---|---|---|
| * bezogen auf insgesamt eingesetztes Propylen | | |
| ** CO/H₂ | | |
| *** n-Butanal:i-Butanal | | |

## Patentansprüche

1. Verfahren zur Herstellung von Aldehyden, dadurch gekennzeichnet, daß man eine olefinische Verbindung mit 2 bis 20 Kohlenstoffatomen mit Kohlenmonoxid und Wasserstoff in Anwesenheit oder Abwesenheit eines Lösungsmittels in Gegenwart eines Katalysators enthaltend Rhodium und eine Verbindung oder ein Gemisch von Verbindungen der allgemeinen Formel (I) 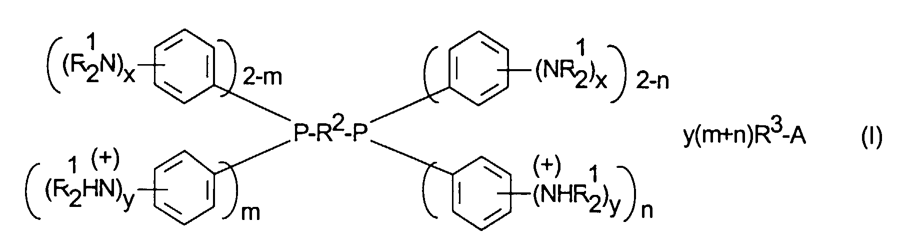 worin R¹ für H oder einen Alkylrest mit 1 bis 12 Kohlenstoffatomen, R² für einen geradkettigen Alkylenrest mit 1 bis 8 Kohlenstoffatomen, einen Sauerstoff enthaltenden Alkylenrest mit 2 bis 4 Kohlenstoffatomen, einen Rest der Formel (II) oder (III) 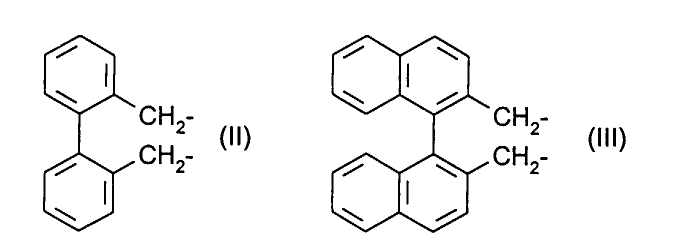 oder einen Cycloalkylenrest mit 3 bis 10 Kohlenstoffatomen, R³ für einen Alkylrest mit 1 bis 25 Kohlenstoffatomen oder einen Arylrest mit 6 bis 10 Kohlenstoffatomen steht, A einen Rest -COO⁻ oder -SO₃⁻ darstellt und x = 0, y = 1, m = 1 und n = 1 ist, oder x = 1, y = 1, m = (1 oder 2) und n = (1 oder 2) ist, oder, falls R² einen Rest der Formel (II) oder (III) darstellt, x = 1, y = 0, m = (0 oder 1) und n = (0 oder 1) ist, bei einer Temperatur von 50 bis 190°C und unter einem Druck von 0,1 bis 45 MPa umsetzt und das Reaktionsprodukt vom Katalysator abtrennt.

2. Verfahren nach Anspruch 1, dadurch gekennzeichnet, daß man eine Verbindung der Formel (I), worin x = 0, y = 1, m = 1 und n = 1 ist oder x = 1, y = 1, m = (1 oder 2) und n = (1 oder 2) ist, oder ein Gemisch dieser Verbindungen einsetzt.

3. Verfahren nach Anspruch 1 oder 2, dadurch gekennzeichnet, daß man eine Verbindung der Formel (I) worin x = 1, y = 1, m = (1 oder 2) und n = (1 oder 2) ist, oder ein Gemisch dieser Verbindungen einsetzt.

4. Verfahren nach einem oder mehreren der Ansprüche 1 bis 3, dadurch gekennzeichnet, daß man eine Verbindung der Formel (I), worin die R¹₂N- und die R¹₂HN⁺-Gruppe in meta- oder para-Stellung, insbesondere in para-Stellung zu der Bindung, die den Benzolring mit dem jeweiligen P-Atom verbindet, steht, oder ein Gemisch dieser Verbindungen einsetzt.

5. Verfahren nach einem oder mehreren der Ansprüche 1 bis 4, dadurch gekennzeichnet, daß man eine Verbindung der Formel (I), worin R¹ für einen Alkylrest mit 1 bis 4 Kohlenstoffatomen, insbesondere für einen Methyl- oder Ethylrest, bevorzugt für einen Methylrest steht, oder ein Gemisch dieser Verbindungen einsetzt.

6. Verfahren nach einem oder mehreren der Ansprüche 1 bis 5, dadurch gekennzeichnet, daß man eine Verbindung der Formel (I), worin R² für einen Alkylenrest mit 1 bis 4 Kohlenstoffatomen oder einen Rest -(CH₂)₂-O-(CH₂)₂-, insbesondere für einen Trimethylen- oder Tetramethylenrest steht, oder ein Gemisch dieser Verbindungen, einsetzt.

7. Verfahren nach einem oder mehreren der Ansprüche 1 bis 6, dadurch gekennzeichnet, daß man eine Verbindung der Formel (I), worin R³ für einen Alkylrest mit 12 bis 24 Kohlenstoffatomen oder einen Arylrest mit 6 bis 7 Kohlenstoffatomen, insbesondere für einen Alkylrest mit 14 bis 22 Kohlenstoffatomen steht, oder ein Gemisch dieser Verbindungen einsetzt.

8. Verfahren nach einem oder mehreren der Ansprüche 1 bis 7, dadurch gekennzeichnet, daß man eine Verbindung der Formel (I), worin A für einen Rest -COO⁻ steht, oder ein Gemisch dieser Verbindungen einsetzt.

9. Verfahren nach einem oder mehreren der Ansprüche 1 bis 8, dadurch gekennzeichnet, daß man die Umsetzung in Gegenwart von 5 bis 500, insbesondere 10 bis 150 ppm Rhodium, bezogen auf olefinische Verbindung durchführt.

10. Verfahren nach einem oder mehreren der Ansprüche 1 bis 9, dadurch gekennzeichnet, daß man die Verbindung der Formel (I) und Rhodium in einem Verhältnis von 1:1 bis 200:1, insbesondere 2:1 bis 10:1 (Mol Verbindung der Formel (I) je g -Atom Rhodium) einsetzt.

11. Verfahren nach einem oder mehreren der Ansprüche 1 bis 10, dadurch gekennzeichnet, daß man als olefinische Verbindung ein substituiertes oder unsubstituiertes Alken mit 2 bis 20 Kohlenstoffatomen, ein substituiertes oder unsubstituiertes Dien mit 4 bis 10 Kohlenstoffatomen, ein substituiertes oder unsubstituiertes Cycloalken oder Dicycloalken mit 5 bis 12 Kohlenstoffatomen im Ringsystem, einen Ester einer ungesättigten Carbonsäure mit 3 bis 20 und eines aliphatischen Alkohols mit 1 bis 18 Kohlenstoffatomen, einen Ester einer gesättigten Carbonsäure mit 2 bis 20 Kohlenstoffatomen und eines ungesättigten Alkohols mit 2 bis 18 Kohlenstoffatomen, einen ungesättigten Alkohol oder ungesättigten Ether mit jeweils 3 bis 20 Kohlenstoffatomen oder ein araliphatisches Olefin mit 8 bis 20 Kohlenstoffatomen einsetzt.

12. Verfahren nach einem oder mehreren der Ansprüche 1 bis 11, dadurch gekennzeichnet, daß man als olefinische Verbindung Dicyclopentadien einsetzt.

13. Verfahren nach einem oder mehreren der Ansprüche 1 bis 12, dadurch gekennzeichnet, daß man die Umsetzung bei einer Temperatur von 80 bis 180°C, insbesondere 100 bis 170°C durchgeführt.

14. Verfahren nach einem oder mehreren der Ansprüche 1 bis 13, dadurch gekennzeichnet, daß man die Umsetzung bei einem Druck von 1 bis 35, insbesondere 3 bis 30 MPa durchführt.

15. Verfahren nach einem oder mehreren der Ansprüche 1 bis 14, dadurch gekennzeichnet, daß man das Reaktionsgemisch mittels Destillation, Extraktion oder Druckfiltration vom Katalysator abtrennt.

16. Verfahren nach einem oder mehreren der Ansprüche 1 bis 15, dadurch gekennzeichnet, daß man das Reaktionsgemisch durch Druckfiltration mittels einer semipermeablen Membran vom Katalysator abtrennt.

17. Verfahren nach Anspruch 16, dadurch gekennzeichnet, daß man als Membran eine Polyamidmembran einsetzt.

18. Verfahren nach Anspruch 16, dadurch gekennzeichnet, daß man als Membran eine Poylaramidmembran einsetzt.

## Claims

1. A process for the preparation of an aldehyde, which comprises reacting an olefinic compound having 2 to 20 carbon atoms with carbon monoxide and hydrogen, in the presence or absence of a solvent, in the presence of a catalyst comprising rhodium and a compound or a mixture of compounds of the formula (I) 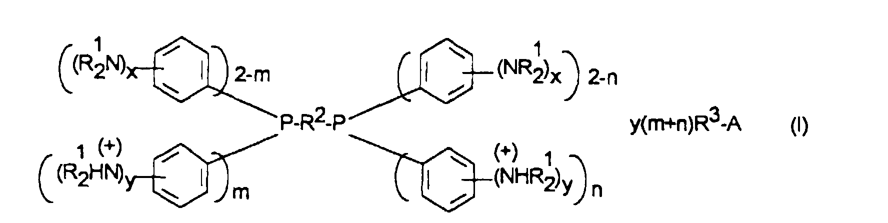 in which R¹ is H or an alkyl radical having 1 to 12 carbon atoms, R² is a straight-chain alkylene radical having 1 to 8 carbon atoms, an oxygen-containing alkylene radical having 2 to 4 carbon atoms, a radical of the formula (II) or (III) 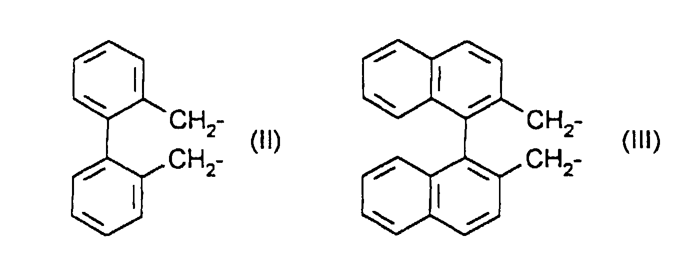 or a cycloalkylene radical having 3 to 10 carbon atoms, R³ is an alkyl radical having 1 to 25 carbon atoms or an aryl radical having 6 to 10 carbon atoms, A is a radical -COO⁻ or -SO₃⁻ and x = 0, y = 1, m = 1 and n = 1, or x = 1, y = 1, m = (1 or 2) and n = (1 or 2), or, if R² is a radical of the formula (II) or (III), x = 1, y = 0, m = (0 or 1) and n = (0 or 1), at a temperature from 50 to 190°C and under a pressure from 0.1 to 45 MPa, and separating the reaction product off from the catalyst.

2. The process as claimed in claim 1, wherein a compound of the formula (I) in which x = 0, y = 1, m = 1 and n = 1, or x = 1, y = 1, m = (1 or 2) and n = (1 or 2), or a mixture of these compounds, is employed.

3. The process as claimed in claim 1 or 2, wherein a compound of the formula (I) in which x = 1, y = 1, m = (1 or 2) and n = (1 or 2), or a mixture of these compounds, is employed.

4. The process as claimed in one or more of claims 1 to 3, wherein a compound of the formula (I) in which the R¹₂N- and the R¹₂HN⁺-group are in the meta- or para-position, in particular in the para-position, relative to the bond which joins the benzene ring to the particular P atom, or a mixture of these compounds, is employed.

5. The process as claimed in one or more of claims 1 to 4, wherein a compound of the formula (I) in which R¹ is an alkyl radical having 1 to 4 carbon atoms, in particular a methyl or ethyl radical, preferably a methyl radical, or a mixture of these compounds, is employed.

6. The process as claimed in one or more of claims 1 to 5, wherein a compound of the formula (I) in which R² is an alkylene radical having 1 to 4 carbon atoms or a radical -(CH₂)₂-O-(CH₂)₂-, in particular a trimethylene or tetramethylene radical, or a mixture of these compounds, is employed.

7. The process as claimed in one or more of claims 1 to 6, wherein a compound of the formula (I) in which R³ is an alkyl radical having 12 to 24 carbon atoms or an aryl radical having 6 or 7 carbon atoms, in particular an alkyl radical having 14 to 22 carbon atoms, or a mixture of these compounds, is employed.

8. The process as claimed in one or more of claims 1 to 7, wherein a compound of the formula (I) in which A is a radical -COO⁻ or a mixture of such compounds, is employed.

9. The process as claimed in one or more of claims 1 to 8, wherein the reaction is carried out in the presence of 5 to 500, in particular 10 to 150, ppm of rhodium, based on the olefinic compound.

10. The process as claimed in one or more of claims 1 to 9, wherein the compound of the formula (I) and rhodium are employed in a ratio of 1:1 to 200:1, in particular 2:1 to 10:1 (mol of compound of the formula (I) per g atom of rhodium).

11. The process as claimed in one or more of claims 1 to 10, wherein a substituted or unsubstituted alkene having 2 to 20 carbon atoms, a substituted or unsubstituted diene having 4 to 10 carbon atoms, a substituted or unsubstituted cycloalkene or dicycloalkene having 5 to 12 carbon atoms in the ring system, an ester of an unsaturated carboxylic acid having 3 to 20 carbon atoms and an aliphatic alcohol having 1 to 18 carbon atoms, an ester of a saturated carboxylic acid having 2 to 20 carbon atoms and an unsaturated alcohol having 2 to 18 carbon atoms, an unsaturated alcohol or ether having in each case 3 to 20 carbon atoms or an araliphatic olefin having 8 to 20 carbon atoms can be employed as the olefinic compound.

12. The process as claimed in one or more of claims 1 to 11, wherein dicyclopentadiene is employed as the olefinic compound.

13. The process as claimed in one or more of claims 1 to 12, wherein the reaction is carried out at a temperature of 80 to 180°C, in particular 100 to 170°C.

14. The process as claimed in one or more of claims 1 to 13, wherein the reaction is carried out under a pressure of 1 to 35 MPa, in particular 3 to 30 MPa.

15. The process as claimed in one or more of claims 1 to 14, wherein the reaction mixture is separated off from the catalyst by means of distillation, extraction or pressure filtration.

16. The process as claimed in one or more of claims 1 to 15, wherein the reaction mixture is separated off from the catalyst by means of a semipermeable membrane.

17. The process as claimed in claim 16, wherein a polyamide membrane is employed as the membrane.

18. The process as claimed in claim 16, wherein a polyaramid membrane is employed as the membrane.

## Revendications

1. Procédé pour la préparation d'aldéhydes caractérisé en ce que l'on fait réagir à une température de 50 à 190°C et sous une pression de 0,1 à 45 MPa un composé oléfinique ayant de 2 à 20 atomes de carbone avec du monoxyde de carbone et de l'hydrogène en présence ou en l'absence d'un solvant, en présence d'un catalyseur contenant du rhodium et un composé ou un mélange de composés de la formule générale (I) 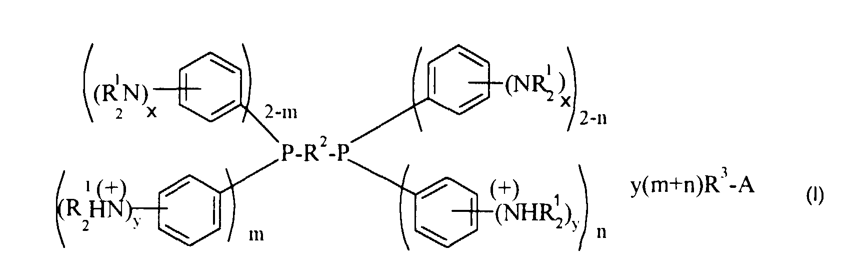 dans laquelle R¹ représente H ou un reste alkyle ayant de 1 à 12 atomes de carbone, R² représente un reste alkylène linéaire ayant de 1 à 8 atomes de carbone, un reste alkylène contenant de l'oxygène ayant de 2 à 4 atomes de carbone, un reste de la formule (II) ou (III) 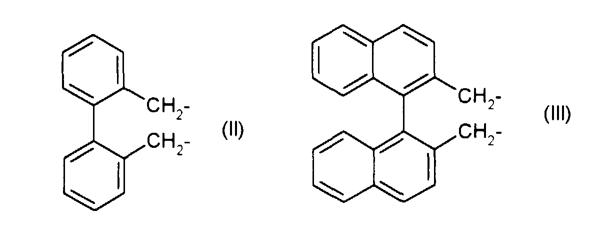 ou un reste cycloalkylène ayant de 3 à 10 atomes de carbone, R³ représente un reste alkyle ayant de 1 à 25 atomes de carbone ou un reste aryle ayant de 6 à 10 atomes de carbone, A représente un reste -COO⁻ ou -SO₃⁻ et x = 0, y = 1, m = 1 et n = 1, ou x = 1, y = 1, m = (1 ou 2) et n = (1 ou 2), ou dans le cas où R² est un reste de la formule (II) ou (III), x = 1, y = 0, m = (0 ou 1) et n = (0 ou 1), et on sépare le produit de la réaction du catalyseur.

2. Procédé selon la revendication 1, caractérisé en ce que l'on utilise un composé de la formule (I), dans laquelle x = 0, y = 1, m = 1 et n = 1 ou x = 1, y = 1, m = (1 ou 2) et n = (1 ou 2), ou un mélange de ces composés.

3. Procédé selon la revendication 1 ou 2, caractérisé en ce que l'on utilise un composé de la formule (1), dans laquelle x = 1, y = 1, m = (1 ou 2) et n = (1 ou 2) ou un mélange de ces composés.

4. Procédé selon l'une ou plusieurs quelconques des revendications 1 à 3, caractérisé en ce que l'on utilise un composé de la formule (1), dans laquelle les groupe R¹₂N et R¹₂HN⁺ sont en position méta ou para, particulièrement en position para par rapport à la liaison qui lie le cycle benzène à l'atome P correspondant, ou un mélange de ces composés.

5. Procédé selon l'une ou plusieurs quelconques des revendications 1 à 4, caractérisé en ce que l'on utilise un composé de la formule (1), dans laquelle R¹ représente un reste alkyle ayant de 1 à 4 atomes de carbone, en particulier un reste méthyle ou éthyle, de préférence un reste méthyle, ou un mélange de ces composés.

6. Procédé selon l'une ou plusieurs quelconques des revendications 1 à 5, caractérisé en ce que l'on utilise un composé de la formule (1), dans laquelle R² représente un reste alkylène ayant de 1 à 4 atomes de carbone ou un reste -(CH₂)₂-O-(CH₂)₂-, en particulier un reste triméthylène ou tétraméthylène, ou un mélange de ces composés.

7. Procédé selon l'une ou plusieurs quelconques des revendications 1 à 6, caractérisé en ce que l'on utilise un composé de la formule (I), dans laquelle R³ représente un reste alkyle ayant de 12 à 24 atomes de carbone ou un reste aryle ayant de 6 à 7 atomes de carbone, en particulier un reste alkyle ayant de 14 à 22 atomes de carbone, ou un mélange de ces composés.

8. Procédé selon l'une ou plusieurs quelconques des revendications 1 à 7, caractérisé en ce que l'on utilise un composé de la formule (1), dans laquelle A représente un reste -COO⁻ ou un mélange de ces composés.

9. Procédé selon l'une ou plusieurs quelconques des revendications 1 à 8, caractérisé en ce que l'on réalise la réaction en présence de 5 à 500, en particulier de 10 à 150 ppm de rhodium, rapportés au composé oléfinique.

10. Procédé selon l'une ou plusieurs quelconques des revendications 1 à 9, caractérisé en ce que l'on utilise le composé de la formule (I) et le rhodium dans un rapport de 1 : 1 à 200 : 1, en particulier de 2 : 1 à 10 : 1 (mol de composé de la formule (I) par atome -g de rhodium).

11. Procédé selon l'une ou plusieurs quelconques des revendications 1 à 10, caractérisé en ce que l'on utilise comme composé oléfinique un alcène substitué ou non substitué ayant de 2 à 20 atomes de carbone, un diène substitué ou non substitué ayant de 4 à 10 atomes de carbone, un cycloalcène ou un dicycloalcène substitué ou non substitué ayant de 5 à 12 atomes de carbone dans le système cyclique, un ester d'un acide carboxylique insaturé ayant de 3 à 20 atomes de carbone et d'un alcool aliphatique ayant de 1 à 18 atomes de carbone, un ester d'un acide carboxylique saturé ayant de 2 à 20 atomes de carbone et d'un alcool insaturé ayant de 2 à 18 atomes de carbone, un alcool insaturé ou un ester insaturé ayant à chaque fois de 3 à 20 atomes de carbone ou une oléfine araliphatique ayant de 8 à 20 atomes de carbone.

12. Procédé selon l'une ou plusieurs quelconques des revendications 1 à 11, caractérisé en ce que l'on utilise comme composé oléfinique le dicyclopentadiène.

13. Procédé selon l'une ou plusieurs quelconques des revendications 1 à 12, caractérisé en ce que l'on réalise la réaction à une température de 80 à 180°C, en particulier de 100 à 170°C.

14. Procédé selon l'une ou plusieurs quelconques des revendications 1 à 13, caractérisé en ce que l'on réalise la réaction à une pression de 1 à 35, en particulier de 3 à 30 MPa.

15. Procédé selon l'une ou plusieurs quelconques des revendications 1 à 14, caractérisé en ce que l'on sépare le mélange réactionnel du catalyseur au moyen d'une distillation, d'une extraction ou d'une filtration sous pression.

16. Procédé selon l'une ou plusieurs quelconques des revendications 1 à 15, caractérisé en ce que l'on sépare le mélange réactionnel du catalyseur par filtration sous pression au moyen d'une membrane semi-perméable.

17. Procédé selon la revendication 16, caractérisé en ce que l'on utilise comme membrane une membrane de polyamide.

18. Procédé selon la revendication 16, caractérisé en ce que l'on utilise comme membrane une membrane de polyaramide.
